# EUROPEAN PATENT SPECIFICATION

(11) **EP 1 433 500 B1**
(45) Date of publication and mention of the grant of the patent: **19.07.2006**
(21) Application number: 03029056.3
(22) Date of filing: 17.12.2003
(51) Int. Cl.: A61P 9/10, A61P 9/12, A61K 36/02, A23L 1/337, A23L 1/30

(54) **Blood fluidity-improving health foods**
Die Blut-Fluidität verbessernde Lebensmittel
Aliments améliorant la fluidité sanguine

(30) Priority: 25.12.2002 JP 2002374170
(43) Date of publication of application: 30.06.2004
(73) Proprietor: SHIRAKO Co., Ltd., Tokyo, 134-8502 (JP)
(72) Inventor: Hagino, Hiroshi, Tokyo, 152-0022 (JP); Saito, Masanobu, Chiba-shi Chiba-ken, 262-0044 (JP)
(74) Representative: HOFFMANN EITLE

(56) References cited:
- EP-A- 0 528 033
- US-A- 4 690 828
- US-B1- 6 217 879
- PATENT ABSTRACTS OF JAPAN vol. 2000, no. 11, 3 January 2001 (2001-01-03) -& JP 2000 226333 A (SHIRAKO:KK), 15 August 2000 (2000-08-15)
- PATENT ABSTRACTS OF JAPAN vol. 017, no. 446 (C-1098), 17 August 1993 (1993-08-17) -& JP 05 103641 A (MARINO FORUM 21), 27 April 1993 (1993-04-27)
- PATENT ABSTRACTS OF JAPAN vol. 006, no. 102 (C-107), 11 June 1982 (1982-06-11) -& JP 57 033579 A (NAKAMURA ATSUSHI), 23 February 1982 (1982-02-23)
- DATABASE WPI Section Ch, Week 200408 Derwent Publications Ltd., London, GB; Class B04, AN 2004-075386 XP002277112 -& JP 2003 304830 A (AKAHO KASEI KK) 28 October 2003 (2003-10-28)
- PATENT ABSTRACTS OF JAPAN vol. 2002, no. 11, 6 November 2002 (2002-11-06) -& JP 2002 187849 A (SAKAMOTO YAKUSOEN:KK), 5 July 2002 (2002-07-05)

## Description

### Technical Field

The present invention relates to blood fluidity-improving health foods capable of improving the fluidity of blood.

### Background Art

Blood plays an important role in maintaining life by transporting oxygen and nutriments and simultaneously transferring waste matter, carbon dioxide etc. from organs to the lung, kidney, liver etc. by circulation through blood vessels in the body.

In recent years, patients with life-style related diseases such as hypertension, hyperlipemia and diabetes are rapidly increasing in Japan because of westernization of eating habits and disturbance in way of life and habits, to cause a social problem. It was revealed that these diseases are related closely to blood fluidity. That is, it is observed that blood fluidity is lowered due to aging, smoking, disturbance in way of life and habits, and the life-style related diseases. A reduction in blood fluidity in micro blood vessels is also noted in diseases in organs in the circulatory system. In addition, it is suggested that the reduction in blood fluidity causes ischemic cerebrovascular disorders, cerebral infarction, pulmonary thromboembolism, myocardial infarction, bleeding in the atrium, mesenteric thrombosis, lower-extremity arteriovenous thrombosis etc.

Conventionally, chemicals such as isoxsuprine hydrochloride, cinnarizine and cinepazide maleate have been used as chemicals for ameliorating easily occurring blood accumulation and micro thrombosis in the minor circulatory system, and many of these chemicals improve the circulation of peripheral blood by expanding blood vessels by their relaxing action on smooth muscles. For improving blood circulation in micro blood vessels, it is important to improve blood fluidity not only by expanding blood vessels with these chemicals but also by improving erythrocyte deformability to reduce blood viscosity. Accordingly, improvements in the fluidity of blood itself are brought into focus while attention is paid to blood rheology in recent years. Examples of improvements in blood fluidity include reports on a mixture of rutin contained in buckwheat, ginkgo leaves and herb medicines (see JP-A 6-172196), hydroxymethyl furfural derivatives (see Japanese Patent No. 2979305), 5-hydroxymethyl furan derivatives (see JP-A 9-216821), γ-linolenic acid alone or γ-linolenic acid and a lipid-soluble antioxidant (see JP-A 10-147523), catechin (see JP-A 10-72460), fish bile and/or its extract with a polar solvent (see JP-A 7-138168), saffron or a tissue culture of saffron (see JP-A 10-287576), a glucosamine salt or a glucosamine salt derivative (see JP-A 2002-97143) and collagen peptides (see Japanese Patent No. 3197547). It is also reported that by giving feed containing unsaturated fatty acids to rats, the deformability of erythrocytes was improved (Johannes M. B. et al.: Thrombosis Research, 81(2), pp. 283-288), or upon ingestion of unsaturated fatty acids by humans, the deformability of erythrocytes was also improved (T. Terano et al.,: Atherosclerosis, 46, pp. 321-331 (1983); I. J. Cartwright et al.: Atherosclerosis, 55, pp. 267-281 (1985); J. B. Hansen et al.: Journal of Internal Medicine, 225, Suppl. 1, pp. 133-139 (1989)).

US 6 217 879 discloses a method of treating laver wherein laver is boiled as a starting material and then treated with pepsin. The resulting material has enhanced antihypertensive action.

JP 2000 226333 discloses the extraction of brown algae by a method that yields water soluble products which can be used to treat diabetic complications.

JP 05 103 641 discloses a degradation product of brown algae obtained by treating starting material with polysaccharide hydrolysing enzyme. The resulting product can be used as an antihypertensive.

JP 57 033 579 discloses the extraction of various seaweeds by boiling and filtering. Again an antihypertensive activity of the extract is disclosed.

EP 0 528 033 relates to de-coloring of Porphyra by treating the starting material with a protease and an organic solvent to obtain white material. No particular medical application is disclosed.

US 4 690 828 describes the preparation of a coagulated food made from brown algae. This document does not disclose any particular medical application.

### Summary of Invention

The object of this invention is to provide a novel health food which is effective in prevention and treatment of life-style related diseases in organs in the circulatory system, such as hypertension, cerebral infarction, myocardial infarction etc. by improving blood fluidity.

The present inventors searched for substances having high safety, capable of being incorporated into a wide variety of general foods and exhibiting an improving effect on blood fluidity, and as a result, they found that seaweeds of the genera Porphyra and seaweeds of the genera Undaria satisfy these conditions, and their active ingredients are phospholipid components of seaweeds of the genera Porphyra or seaweeds of the genera Undaria, and this invention was thereby made.

That is, this invention relates to a blood fluidity-improving health food comprising phospholipid components of seaweeds of the genera Porphyra and/or seaweeds of the genera Undaria.

### Brief Description of Drawing

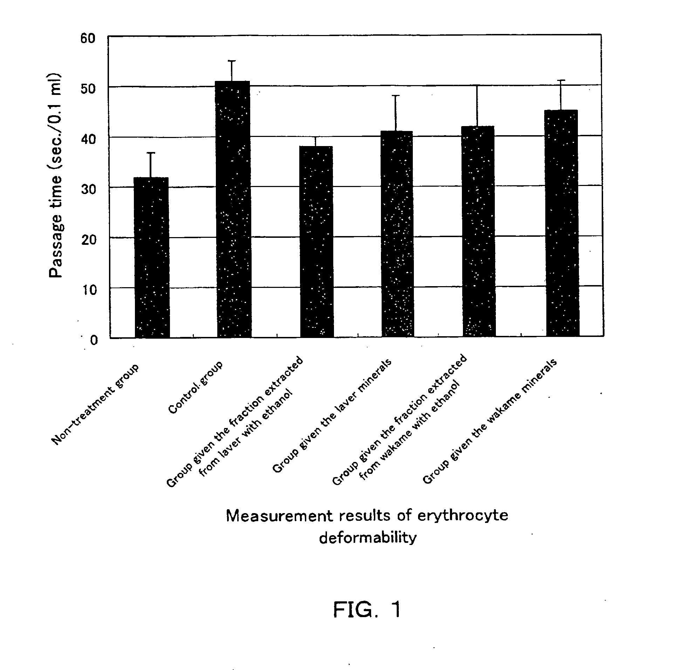

Fig. 1 is a graph showing the results of an erythrocyte deformability test using rats.

### Disclosure of Invention

The fluidity of blood is influenced by physical characteristics of blood cells, such as erythrocyte deformability, leukocyte deformability and adherence, and platelet aggregation ability. For example, erythrocyte is deformed so as to pass through a blood vessel having a diameter smaller than its diameter. This ability to be deformed is called deformability. Normal erythrocyte has a diameter of about 8 µm, but can easily pass without disruption through a vessel as very thin as about 3 µm in diameter because of its deformability. Such deformability plays a very important role in passing through a thin capillary vessel.

The present inventors found that blood fluidity was improved by administering seaweeds of the genera Porphyra or seaweeds of the genera Undaria into rats whose blood fluidity had been significantly reduced by administering a high-fat food. Upon administration of the seaweeds, promotion of erythrocyte deformability and reduction in platelet aggregation were observed, and a change in formulation of fatty acids present in erythrocyte membranes was confirmed. Specifically, an increase in linoleic acid, a decrease in arachidonic acid and an increase in eicosapentaenoic acid in the formulation of fatty acids in rat erythrocyte membranes were observed. In addition, it was found by analysis of mineral components in rat blood that major mineral components such as calcium, magnesium etc. in seaweeds of the genera Porphyra or seaweeds of the genera Undaria were absorbed into blood.

The present inventors made extensive study, and as a result, they found that seaweeds of the genera Porphyra or seaweeds of the genera Undaria themselves have an improving effect on blood fluidity, and that the seaweeds can further improve the effect by extracting phospholipid components therefrom, and can be utilized as health food having a significant improving effect on blood fluidity.

The seaweeds of the genera Porphyra or the seaweeds of the genera Undaria used in the present invention, including those generally eaten by us, may be in any forms such as raw, dried or salted forms or in the form of dry powder, or of any varieties.

To extract phospholipid components from these seaweeds, generally used extraction methods such as solvent extraction, supercritical extraction etc. are used. In experiments conducted by the present inventors, the phospholipid components could be efficiently extracted with a water-soluble organic solvent such as ethanol or a water-soluble organic solvent such as water-containing ethanol.

As described above, the phospholipid components from the seaweeds of the genera Porphyra or the seaweeds of the genera Undaria are added in a predetermined amount to food materials and processed in a usual production method, to give blood fluidity-improving nutrient drinks, nutrient assistant foods and health foods endowed with blood fluidity-improving functions. In addition in the phospholipid components mineral components may be used. The amount thereof in foods is not particularly limited. Because the seaweeds of the genera Porphyra or the seaweeds of the genera Undaria are originally highly safe food materials, their extracted components can also be incorporated in a wide range.

### Best Mode for Carrying Out the Invention

Hereinafter, embodiments of this invention are described. This invention is not limited to the following examples.

### Example 1. Preparation of phospholipids from dry laver

Dry laver was milled into powder of about 50-mesh size with a high-speed mill and then extracted with 20 L of 60% ethanol under stirring at ordinary temperature for 3 hours. The material was separated by centrifugation into dry seaweed powder and an ethanol extract. The fraction extracted with ethanol was concentrated under reduced pressure to remove the ethanol.

This ethanol fraction was analyzed for formulation of fatty acids in the phospholipid components. The results are shown in Table 1.

**Table 1**

| Fatty acid | (mol%) |
|---|---|
| Myristic acid | 1.21 |
| Palmitic acid | 19.22 |
| Stearic acid | 1.43 |
| Oleic acid | 5.01 |
| Linoleic acid | 3.69 |
| γ-Linolenic acid | 2.24 |
| Icosenoic acid | 6.59 |
| Icosadienoic acid | 1 |
| Arachidonic acid | 5.77 |
| Eicosapentanoic acid | 53.84 |

### Comparative Example 1. Preparation of mineral components from dry layer

5 kg of the laver powder used in Example 1 was converted into ashes under strong heating at 550°C, to give 986 g mineral components of the laver. The mineral components were subjected to elemental analysis. The results are shown in Table 2.

**Table 2.**

| Element | (mg%) |
|---|---|
| Calcium | 440.0 |
| Phosphorus | 650.0 |
| Iron | 13.0 |
| Sodium | 625.0 |
| Potassium | 3100.0 |
| Manganese | 4.0 |
| Zinc | 10.0 |
| Copper | 1.5 |
| Iodine | 0.1 |
| Selenium | 0.1 |
| Magnesium | 270.0 |

### Example 2. Preparation of phospholipids from dry layer

Dry wakame seaweed was milled into powder of about 50-mesh size with a high-speed mill and then extracted with 200 ml of 60% ethanol under stirring at ordinary temperature for 3 hours. The material was separated by centrifugation into dry wakame seaweed powder and an ethanol extract. The ethanol extract was concentrated under reduced pressure to remove the ethanol.

This fraction extracted with ethanol was analyzed for formulation of fatty acids in the phospholipid components. The results are shown in Table 3.

**Table 3**

| Fatty acid | (mol%) |
|---|---|
| Myristic acid | 9.64 |
| Palmitic acid | 25.17 |
| Stearic acid | 1.92 |
| Oleic acid | 10.54 |
| Linoleic acid | 13.21 |
| γ-Linolenic acid | 3.45 |
| Icosenoic acid | 10.7 |
| Icosadienoic acid | 0.27 |
| Arachidonic acid | 14.3 |
| Eicosapentanoic acid | 10.8 |

### Comparative Example 2. Preparation of mineral components from dry wakame seaweed

20 g of the wakame seaweed powder used in Example 2 was converted into ashes under strong heating at 550°C, to give 1.9 g mineral components of the wakame seaweed. The mineral components were subjected to elemental analysis. The results are shown in Table 4.

**Table 4**

| Element | (mg%) |
|---|---|
| Calcium | 960.0 |
| Phosphorus | 400.0 |
| Iron | 5.4 |
| Sodium | 6100.0 |
| Potassium | 5500.0 |
| Manganese | 0.6 |
| Zinc | 2.0 |
| Copper | 0.3 |
| Iodine | 7.8 |
| Selenium | 0.1 |
| Magnesium | 1172.0 |

### Comparative Test Example 1. Measurement of the fluidity of whole blood

Two to three hours after breakfast, blood was collected via a median cubitus vein from a 56-years-old healthy person sitting at rest, and 5% heparin (1000 U/ml) was added to the obtained fresh blood. Then, the obtained blood was pipetted in an amount of 500 sample. The mineral components obtained in Comparative Example 1 or 2 were diluted 20-fold with physiological saline, and 5 µl of the dilution was added to a supernatant of each sample. 5 µl physiological saline was added to the control group. The fluidity of each whole blood was measured with a cytomicrorheology measuring instrument MC-FAN (Hitachiharamachi Denshi Kogyo Co., Ltd.). The results are shown in Table 5. As can be seen from the results, the mineral components obtained from each of the dry laver and dry wakame seaweed were confirmed to exhibit an excellent blood fluidity-improving action.

**Table 5**

| | Passage time of whole blood |
|---|---|
| Control group | 62 seconds |
| Addition of the 20-fold dilution of the laver mineral components | 41 seconds |
| Addition of the 200-fold dilution of the laver mineral components | 52 seconds |
| Addition of the 20-fold dilution of the wakame mineral components | 47 seconds |
| Addition of the 200-fold dilution of the wakame mineral components | 53 seconds |

### Comparative Example 3

The mineral components obtained in Comparative Example 1 were used to produce a health food in the form of tablets. 100 g of the mineral components from the dry seaweed, 50 g reducing maltose syrup, 1 g citric acid and 0.5 g perfume were well mixed and tabletted into tablets (300 mg/tablet) as health food.

### Comparative Test Example 2

The object of clinical examination was 12 healthy men in their forties to fifties. They were examined for the passage time of their whole blood in the same manner as in Test Example 1 and divided into two groups such that the two groups have almost the same passage time. Six tablets (about 1.8 g) obtained in Example 5 were ingested by one group every day for 1 week. The other group was instructed not to take processed foods of laver and wakame seaweed. Both the groups were instructed not to change their way of life. One week thereafter, they were examined again for the passage time of their whole blood. The results are shown in Table 6. As can be seen from the results, an excellent blood fluidity-improving effect by daily administration of 6 tablets obtained in Comparative Example 3 was recognized.

**Table 6**

| | | |
|---|---|---|
| | Just before the test | after 1 week |
| Control group | 53.0±4.47 | 53.2±4.71 |
| Group given the layer minerals | 52.8±6.24 | 46.7±3.56 |

### Test Example 1. Test of improvement of erythrocyte deformability in rats

4-Weeks-old male Wister rats were preliminarily bred for 1 week as experimental animals, then divided into groups each consisting of 5 animals and bred for 2 weeks in this experimental. The non-administration group was continuously given MF powdery feed. The test group was given lipid-containing feed containing 5% olive oil, 5% margarine, 1% cholesterol and 1% cholic acid as fundamental feed to which the fraction extracted with ethanol or the mineral components obtained in each of Examples 1 or 2 or comparatives of examples 1 or 2 had been added. The control was given only the fundamental feed.

The feed was exchanged every day, and the animals were allowed drinking water ad libitum. An animal breeding chamber was maintained at room temperature (25°C) in 50±5% humidity in a bright (12 hours)/dark (12 hours) cycle (lighting at 8 AM and light out at 8 PM). The animals were bred for 2 weeks and fasted for 18 hours, the abdomen was opened under ether anesthesia, and from the heart, the blood was collected in a sodium citrate solution such that the final concentration of sodium citrate was 3.8%. The blood was centrifuged (1200×g, 10 minutes) to remove a supernatant, and the erythrocytes were washed with a phosphate buffer. The deformability of the resulting erythrocytes was measured, and the formulation of fatty acids in the resulting erythrocyte membranes was analyzed. The results are shown in Fig. 1 and Table 7.

The deformability of the erythrocytes in the control group, as compared with those of the group not given lipid, was confirmed to be significantly lowered. On the other hand, the groups given the phospholipid-containing fraction extracted with ethanol or the mineral fraction obtained in Examples 1 or 2 or comparatives of examples 1 or 2 as compared with the control group, were confirmed to significantly promote erythrocyte deformability. Further, a change in the formulation of fatty acids in the erythrocyte membrane was observed in the group given the phospholipid components.

**Table 7**

| | Non-administration group | Control group | Group given the fraction extracted from the laver with ethanol | Group given the fraction extracted from the wakame with ethanol |
|---|---|---|---|---|
| Myristic acid | 3.54 | 4.77 | 3.55 | 2.87 |
| Palmitic acid | 40.23 | 37.20 | 38.23 | 33.95 |
| Stearic acid | 8.99 | 7.33 | 7.64 | 7.55 |
| Oleic acid | 7.55 | 12.43 | 10.01 | 14.12 |
| Linoleic acid | 5.98 | 9.01 | 8.54 | 9.22 |
| γ-Linolenic acid | 0.77 | 0.55 | 0.80 | 0.84 |
| α-Linolenic acid | 1.01 | 1.77 | 1.94 | 1.23 |
| Icosenoic acid | 0.99 | 1.23 | 1.44 | 1.34 |
| Icosadienoic acid | 0.47 | 0.71 | 0.44 | 0.61 |
| Arachidonic acid | 24.25 | 19.22 | 17.85 | 22.05 |
| Eicosapentaenoic acid | 0.99 | 1.02 | 4.11 | 1.34 |
| Docosatetraenoic acid | 3.11 | 1.88 | 2.88 | 1.92 |
| Docosahexaenoic acid | 1.11 | 1.66 | 1.22 | 1.43 |
| Hexacosanoic acid | 1.01 | 1.22 | 1.35 | 1.53 |

### Example 3.

The fraction extracted with ethanol obtained in Example 1 was used to prepare a dressing. That is, 15 g of the fraction extracted from the seaweed with ethanol, 0.2 g xanthane gum, 45 g water, 30 g salad oil, 3 g flavor seasoning, 1.4 vinegar, 2.5 g sorbitol, 1.5 g common salt, 0.1 g starch, 0.5 g sodium glutamate, 0.5 g sucrose fatty ester and 0.3 g gum arabic were mixed to prepare a dressing.

### Industrial Applicability

The health food of this invention is useful for improving blood fluidity and can be used for treatment and prevention of hypertension, ischemic cerebrovascular disorders, cerebral infarction, pulmonary thromboembolism, myocardial infarction, bleeding in the atrium, mesenteric thrombosis, lower-extremity arteriovenous thrombosis etc. The health food of this invention is highly safe and can be added widely to general foods because the starting material thereof is seaweed of the genus Porphyra or Undaria.

## Claims

1. Phospholipid extract of seaweeds of the genus Porphyra and/or seaweeds of the genus Undaria.

2. The phospholipid extract according to claim 1, wherein said phospholipid extract is obtainable by extracting seaweeds of the genus Porphyra and/or seaweeds of the genus Undaria with ethanol.

3. Health food for improving blood fluidity, comprising the phospholipid extract according to claim 1 or 2.

4. Use of the phospholipid extract according to claim 1 or 2, or the health food according to claim 3 as a food additive.

## Patentansprüche

1. Phospholipidextrakt von Seegräsern des Genus Porphyra und/oder Seegräsern des Genus Undaria.

2. Phospholipidextrakt gemäß Anspruch 1, wobei dieser Phospholipidextrakt durch Extrahieren von Seegräsern des Genus Porphyra und/oder Seegräsern des Genus Undaria mit Ethanol erhältlich ist.

3. Gesundheitsnahrung für die Verbesserung der Fließfähigkeit des Blutes, umfassend den Phospholipidextrakt gemäß Anspruch 1 oder 2.

4. Verwendung des Phospholipidextraktes gemäß Anspruch 1 oder 2 oder der Gesundheitsnahrung gemäß Anspruch 3 als Nahrungsmittelzusatz.

## Revendications

1. Extrait phospholipidique d'algues du genre Porphyra et/ou d'algues du genre Undaria.

2. Extrait phospholipidique selon la revendication 1, dans lequel ledit extrait phospholipidique est susceptible d'être obtenu par extraction des algues du genre Porphyra et/ou des algues du genre Undaria avec de l'éthanol.

3. Aliment naturel pour améliorer la fluidité du sang, comprenant l'extrait phospholipidique selon la revendication 1 ou 2.

4. Utilisation de l'extrait phospholipidique selon la revendication 1 ou 2, ou de l'aliment naturel selon la revendication 3 en tant qu'additif alimentaire.
